(19) Europäisches Patentamt
European Patent Office
Office européen des brevets

(11) Veröffentlichungsnummer: **0 271 099 A2**

# (12) EUROPÄISCHE PATENTANMELDUNG

(21) Anmeldenummer: **87118322.4**

(22) Anmeldetag: **10.12.87**

(51) Int. Cl.⁴: **C07C 103/50**, C07D 213/81, A61K 31/16, C07D 209/10, C07D 307/82, C07D 307/16, C07D 333/24, C07D 333/60

(30) Priorität: **12.12.86 DE 3642497**

(43) Veröffentlichungstag der Anmeldung: **15.06.88 Patentblatt 88/24**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Kunstmann, Rudolf, Dr.**
**Schlesierstrasse 9**
**D-8901 Aystetten(DE)**
Erfinder: **Hock, Franz, Dr.**
**Altstadt 19**
**D-6110 Dieburg(DE)**
Erfinder: **Wiemer, Gabrielle, Dr.**
**Ostring 88**
**D-6231 Schwalbach am Taunus(DE)**

(54) **Substituierte Aminopropionsäureamide, Verfahren zu ihrer Herstellung, diese enthaltende Mittel und ihre Verwendung sowie die bei der Herstellung anfallenden neuen Zwischenprodukte.**

(57) Aminopropionsäureamide der Formel I

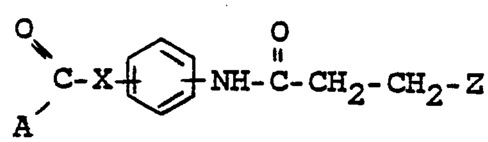

(I)

in der A, X und Z die angegebenen Bedeutungen haben sowie deren physiologisch verträgliche Salze, Verfahren zu ihrer Herstellung und Verwendung derselben als Arzneimittel, in denen diese Verbindungen insbesondere nootrop wirken.

EP 0 271 099 A2

Beschreibung

Substituierte Aminopropionsäureamide, Verfahren zu ihrer Herstellung, diese enthaltende Mittel und ihre Verwendung sowie die bei der Herstellung anfallenden neuen Zwischenprodukte

Bei der Behandlung von Psychosyndromen, wie sie beispielsweise bei der senilen Demenz auftreten, werden neben bestimmten Indolalkaloiden besonders auch Derivate des Pyrrolidon-5 eingesetzt. Eine Reihe jüngster Veröffentlichungen (EP 0045602 (Indolalkaloide); EP 0164853, EP 0170460, EP 0164852, EP 0165919, EP 0089900, EP 0163260 (Pyrrolidon-5-Derivate); EP 0161017 (Peptide)) zeigen die zunehmende Bedeutung des Gebietes einerseits und die enge Begrenzung auf diese drei Verbindungsklassen andererseits.

Abweichend von diesen bekannten Verbindungsklassen wurde nun überraschenderweise gefunden, daß bestimmte Aminopropionsäureamide in Tierversuchen nootrope Eigenschaften zeigen und deshalb für die Behandlung kognitiver Dysfunktionen, wie der senilen Demenz, der Alzheimerschen Krankheit und anderer vergleichbarer Psychosyndrome eingesetzt werden können.

Die Erfindung betrifft daher Aminopropionsäurederivate der Formel I

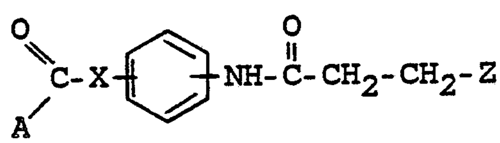

(I)

in der

A Hydroxy, Amino, Alkyl- und Dialkylamine mit jeweils 1-4 C-Atomen im Alkylteil, Alkoxy mit 1-4 C-Atomen im Alkylteil, wobei die genannten Alkylgruppen geradkettig

oder verzweigt sein können,

X Alkylen mit 1-4 C-Atomen oder Alkenylen mit 2-4 C-Atomen, wobei die genannten Alkylgruppen geradkettig oder verzweigt sein können und

Z eine Aminogruppe der Formel II

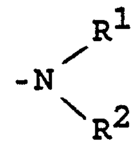

(II)

ist, worin

$R_1$ Wasserstoff und

$R_2$ Aryl-$C_1$-$C_4$-alkyl, wobei die Alkylgruppe geradkettig oder verzweigt ist und Aryl, Phenyl, Pyridyl, Indolyl, Benzofuranyl, Benzothienyl, Pyrrolyl, Furanyl oder Thienyl bedeutet, wobei die Arylreste mit Halogen, Methyl oder 1-, 2- oder 3-fach halogensubstituiertem Methyl mono- oder disubstituiert sein können, bedeutet

oder worin $R_1$ und $R_2$ zusammen mit dem Stickstoffatom einen gesättigten oder ungesättigten 5- oder 6-gliedrigen heterocyclischen Ring bilden, der mit Phenyl oder Phenyl-alkyl mit 1-4 C-Atomen im Alkylteil substituiert ist, wobei die Phenylreste ihrerseits mit Halogen, Methyl oder 1-, 2- oder 3-fach halogensubstituiertem Methyl mono- oder disubstituiert sein können, wobei im Falle des 6-gliedrigen Ringes der Phenyl- oder Phenyl-$C_1$-$C_4$-alkylrest über ein zweites, im 6-Ring befindliches Stickstoffatom gebunden sein kann

sowie deren physiologisch verträglichen Salze.

Bevorzugt sind Verbindungen der Formel I, worin

A Hydroxy, Amino, Alkoxy mit 1-4 C-Atomen im Alkylteil, wobei die genannte Alkygruppe geradkettig oder

verzweigt sein kann,

X Alkylen mit 1-4 C-Atomen oder Alkenylen mit 2 C-Atomen, wobei die genannte Alkylgruppe geradkettig oder verzweigt sein kann,

Z eine Aminogruppe der Formel II ist, worin

$R_1$ Wasserstoff und

$R_2$ Aryl-$C_1$-$C_4$-Alkyl, wobei die Alkylgruppe geradkettig oder verzweigt ist und Aryl, Phenyl, Pyridyl, Indolyl, Benzofuranyl, Benzothienyl, Pyrrolyl, Furanyl oder Thienyl bedeutet, wobei die Arylreste mit Fluor, Chlor, Brom, Methyl oder Trifluormethyl monosubstituiert sein können, bedeutet

oder worin $R_1$ und $R_2$ zusammen mit dem Stickstoffatom einen gesättigten oder ungesättigten 5- oder 6-gliedrigen heterocyclischen Ring bilden, der mit Phenyl oder Phenylalkyl mit 1-4 C-Atomen im Alkylteil substituiert ist, wobei die Phenylreste ihrerseits mit Fluor, Chlor, Brom, Methyl oder Trifluormethyl monosubstituiert sein können und wobei, im Falle des 6-gliedrigen Ringes, der Phenyl- oder Phenyl-$C_1$-$C_4$-alkylrest über ein zweites, im 6-Ring befindliches Stickstoffatom gebunden sein kann

sowie deren physiologisch verträglichen Salze.

Besonders bevorzugt sind Verbindungen der Formel Ia,

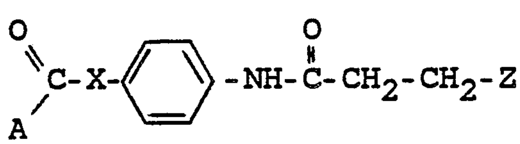

Ia

in der

A Hydroxy,

X Methylen oder Ethylen,

Z eine Aminogruppe der Formel II ist, worin

$R_1$ Wasserstoff

$R_2$ Aryl-$C_1$-$C_3$-alkyl, wobei die Alkylkette geradkettig ist und Aryl, Phenyl oder Indolyl bedeutet, wobei die Phenylreste gegebenenfalls mit Chlor oder Trifluormethyl monosubstituiert sind, bedeutet

oder worin $R_1$ und $R_2$ zusammen mit dem Stickstoffatom einen gesättigten oder 1-fach ungesättigten, 6-gliedrigen Ring bilden, der gegebenenfalls in 4-Stellung ein zweites, im Ring befindliches Stickstoffatom besitzt und in 4-Stellung mit Phenyl substituiert ist, wobei dieser Phenylrest seinerseits mit Chlor oder Trifluormethyl monosubstituiert sein kann

sowie deren physiologisch verträglichen Salze.

Die Erfindung betrifft weiterhin ein Verfahren zur Herstellung der Verbindungen der Formel I, das dadurch gekennzeichnet ist, daß man

a) Amine der Formel III

$$H - Z \qquad (III)$$

oder deren quaternäre Salze,

wobei Z die zu Formel I angegebene Bedeutung hat, mit Acrylsäureaniliden der Formel IV

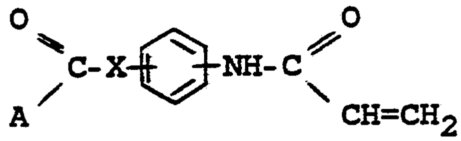

(IV)

worin A und X die zu Formel I angegebenen Bedeutungen haben und worin, falls A Hydroxy ist, die vorhandene freie Carboxyfunktion gegebenenfalls geschützt ist, umsetzt oder

b) das Amin der Formel III mit einem Propionsäureanilid der Formel V

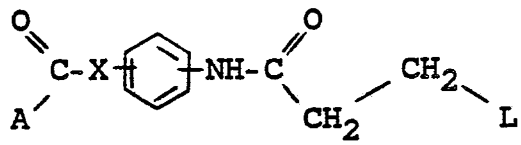

(V)

worin A und X die zu Formel I angegebenen Bedeutungen haben und L einen durch nukleophile Substitution ersetzbaren Rest darstellt und worin, falls A Hydroxy ist, die vorhandene freie Carboxyfunktion gegebenenfalls geschützt ist, umsetzt oder

c) Aminopropionsäurederivate der Formel VI

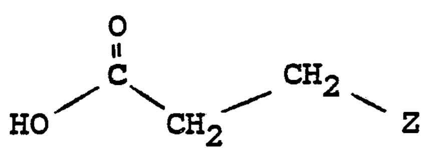

(VI)

worin Z die zu Formel I angegebenen Bedeutungen hat

mit Anilinderivaten der Formel VII

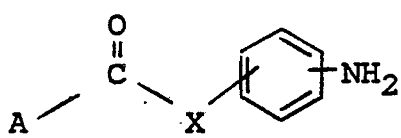

(VII)

umsetzt, wobei A und X die zu Formel I angegebenen Bedeutungen haben und worin, falls A Hydroxy ist, die

vorhandene freie Carboxyfunktion gegebenenfalls geschützt ist und in den nach a), b) oder c) erhaltenen Verbindungen die gegebenenfalls vorhandene Schutzgruppe unter Bildung der freien Carboxylfunktion hydrolytisch oder hydrogenolytisch abspaltet und die Reaktionsprodukte gegebenenfalls in ihre physiologisch verträglichen Salze überführt.

Im folgenden wird die Herstellung von Verbindungen gemäß Formel I und die Herstellung der dafür benötigten Ausgangssubstanzen näher beschrieben.
Als temporäre Carboxylschutzgruppen sowohl für Verfahrensvariante a), b) als auch für c) sind Esterschutzgruppen geeignet, wie sie auch in der Peptidsynthese Anwendung finden (vergleiche z.B. Kontakte Merck 3/79, Seiten 15 und 19 ff).
Häufig verwendet werden die Methyl-, Benzyl- oder tert.-Butylester, ferner ONbzl, OMbzl, OPic. Die Abspaltung erfolgt je nach Schutzgruppe durch saure oder alkalische Hydrolyse oder durch Hydrierung in Gegenwart eines Übergangsmetall-Katalysators (Houben-Weyl, Methoden der organischen Chemie, Band E5, Seiten 496-504, vierte Auflage, 1985).

Die Herstellung der erfindungsgemäßen Verbindungen nach Verfahrensvariante a) gelingt am einfachsten dadurch, daß die beiden Komponenten, das Acrylsäureanilid IV und das entsprechende Amin III in äquimolaren Mengen oder bis zu einem etwa 5-fachen Überschuß an III vermengt werden und bei Temperaturen zwischen 50 und 210° C, bevorzugt bei 110-190° C bis zur Beendigung der Reaktion erhitzt werden. Die Beendigung der Reaktion läßt sich mittels Dünnschichtchromatographie (DC-Kontrolle) bestimmen. Eine Variante dieses Verfahrens besteht darin, daß man in einem geeigneten Lösungsmittel, wie Diethylether oder Dimethoxyethan oder Tetrahydrofuran, Alkoholen, wie Methanol, Ethanol oder Methylglykol, chlorierten Kohlenwasserstoffen, wie Methylenchlorid, Chloroform, Tri-

oder Tetrachlorethylen, Toluol oder auch polaren Lösungsmitteln wie Dimethylformamid oder Dimethylsulfoxid arbeitet. Auch hier kann ein Überschuß von Amin III, der bis zur etwa 5-fachen Menge betragen kann, angewandt werden. Die Reaktionstemperaturen liegen dabei zwischen Raumtemperatur und dem Siedepunkt des Lösungsmittels, wobei Temperaturen im Bereich von 50 bis 130° C besonders bevorzugt sind.

Die Ausgangsverbindungen der Formel III können, soweit sie nicht käuflich sind, einfach synthetisiert werden (z.B. Organikum, Organisch-chemisches Grundpraktikum, 4. Auflage, VEB Deutscher Verlag der Wissenschaften, 1964), eine Übersicht über die verschiedenen Möglichkeiten findet sich im Methodenregister (Beilage zum Organikum, Seite 4). Die Acrylsäureanilide der Formel IV können beispielsweise durch Umsetzung von Acrylsäure mit den entsprechenden carboxygeschützten Anilinderivaten hergestellt werden. Dies kann z.B. mittels Dicyclohexylcarbodiimid in Gegenwart eines polaren Lösungsmittels, wie Dimethylformamid oder Dimethylsulfoxid erfolgen oder über einen aktivierten Ester, wie Chlorameisensäureethylester, d.h. über ein gemischtes Anhydrid. Entsprechende Methoden sind z.B. in Houben-Weyl Band XV/2, Seiten 103-111 (Verwendung von Dicyclohexylcarbodiimid), Seiten 169-183 (gemischte Anhydridmethode) oder in Schröder, Lübke, The Peptides, Vol. 1, Academic press, New York 1965, Seiten 76-136 beschrieben. Am einfachsten gestaltet sich die Synthese dann, wenn man statt der Acrylsäure das entsprechende Säurechlorid einsetzt und dieses nach gängiger Methode mit den gegebenenfalls carboxygeschützten Aniliden zu den Verbindungen der Formel IV umsetzt (vgl. Organikum, Org.-chem. Grundpraktikum, 4. Auflage, VEB Deutscher Verlag der Wissenschaften, 1964, S. 377 ff.).

Bei der Verfahrensvariante b) geht man am einfachsten so vor, daß man geeignet substituierte Propionsäureanilide V, worin

A und X wie oben definiert sind und L für einen Substituenten wie beispielsweise Chlor, Brom, Jod, Acetoxy, Mesyloxy, Tosyloxy oder einen sonst leicht durch nucleophile Substitution ersetzbaren Substituenten steht, entweder in äquimolaren Mengen oder bis in einem etwa 5-fachen Überschuß mit dem Amin III, im Falle der Oxyverbindungen ohne, bei den halogensubstituierten Verbindungen mit oder ohne zusätzlichen Basenzusatz, mit oder ohne Lösungsmittel umsetzt. Als zusätzliche Basen kommen anorganische Säurefänger wie Carbonate oder Hydrogencarbonate z.B. Natrium- oder Kaliumcarbonat oder Natrium- oder Kaliumhydrogencarbonat, oder organische Säurefänger wie tertiäre Amine wie Triethylamin oder Ethyldiisopropylamin in Betracht.

Verbindungen gemäß Formel V lassen sich beispielsweise durch Umsetzung von Propionsäuren, die in ß-Stellung eine gute Abgangsgruppe wie Chlor, Brom, Jod, Acetoxy, Mesyloxy oder Tosyloxy besitzen mit Anilinderivaten der Formel VII unter Zusatz eines wasserabspaltenden Mittels wie Dicyclohexylcarbodiimid (DCC) in einem polaren Lösungsmittel wie Dimethylformamid (DMF) oder Dimethylsulfoxid (DMSO), herstellen. Eine andere Möglichkeit bietet die Umsetzung von Propionsäureestern bzw. - Anhydriden, die wie oben beschrieben in ß-Stellung substituiert sind, mit den Anilinderivaten gemäß Formel VII. Entsprechende Methoden sind z.B. in Houben-Weyl Band XV/2, Seite 103-111 (Verwendung von DCC), Seite 169-183 (gemischte Anhydridmethode) oder in Schröder, Lübke, The Peptides, Vol. 1, Academic Press New York 1965, Seiten 76-136 beschrieben. Statt der β-substituierten Propionsäuren lassen sich auch deren Säurechloride einsetzten, die nach gängiger Methode zu den entsprechenden Aniliden umgesetzt werden (vgl.Organikum, Organisch-chemisches Grundpraktikum, 4. Auflage, VEB Deutscher Verlag der Wissenschaften, 1964, S. 377 ff.).

Die ß-substituierten Propionsäuren, Propionsäurechloride, Propionsäureester oder Propionsäureanhydride erhält man - sofern sie nicht käuflich sind - beispielsweise durch

Umsetzung von ß-Hydroxy-propionsäurederivaten mit Tosylaten, Mesylaten oder Säurehalogeniden.

Bei der Kopplung der Aminosäure VI mit einem Anilinderivat VII gemäß Verfahrensvariante c) kommt beispielsweise der Einsatz von Dicyclohexylcarbodiimid (DCC) als wasserabspaltendes Agens in Frage, wobei als Lösungsmittel ein polares Lösungsmittel wie Dimethylformamid (DMF) oder Dimethylsulfoxid (DMSO) eingesetzt wird oder die Synthese über einen aktivierten Ester, wie beispielsweise die Umsetzung mit Chlorameisensäureethylester zu einem gemischten Anhydrid und anschließende Umsetzung mit dem Anilin zu Verbindungen der Formel I. Entsprechende Methoden sind z.B. in Houben-Weyl Band XV/2 Seite 103-111 (Verwendung von DCC), Seite 169-183 (gemischte Anhydridmethode) oder in Schröder, Lübke, The Peptides, Vol. 1, Academic Press New York 1965, Seiten 76-136 beschrieben.
Zur Herstellung der Ausgangsverbindungen der Formel VI werden Amine der oben definierten Formel III mit Acrylsäureestern der Formel VIII

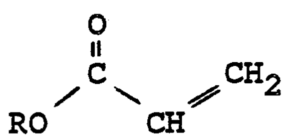

(VIII)

worin R eine Alkylgruppe mit vorzugsweise 1 bis 4 C-Atomen oder eine hydrogenolytisch oder sauer leicht abspaltbare Carboxylschutzgruppe bedeutet, zu Estern der Formel IX

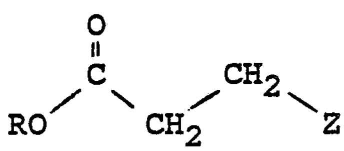

(IX)

worin Z und R wie oben definiert sind, umgesetzt. Man führt die Reaktion entweder mit oder ohne Lösungsmittel durch. Die Temperaturen variieren dabei gewöhnlich zwischen Raumtemperatur und dem Siedepunkt des Reaktionsgemisches

(etwa bis zu 180° C Celsius). Als Lösungsmittel kommen hier wiederum in Betracht: Ether wie Diethylether, Tetrahydrofuran, Dimethoxyethan, Alkohole wie Methanol, Ethanol, Methylglykol, chlorierte Kohlenwasserstoffe wie Methylenchlorid, Chloroform, Tri- oder Tetrachlorethylen oder aromatische Lösungsmittel wie Toluol oder auch polare Lösungsmittel wie Dimethylformamid und Dimethylsulfoxid. Das so erhaltenen Rohprodukt, der 3-Aminopropionsäureester der Formel IX, kann - sofern mit Lösungsmitteln gearbeitet wurde - gegebenenfalls nach Eindampfen des Lösungsmittels, direkt für die Verseifung zu den 3-Aminopropionsäuren VI eingesetzt werden. Man geht dabei am einfachsten so vor, daß man den Ester in einem Gemisch aus Wasser und einem Alkohol wie Methanol oder Ethanol in Gegenwart eines Alkalihydroxids wie z.B. NaOH bis zur Beendigung der Esterspaltung (Kontrolle mittels Dünnschichtchromatographie) bei Temperaturen zwischen Raumtemperatur und dem Siedepunkt des Reaktionsgemisches hält. Ein Temperaturbereich zwischen 40 und 65° C hat sich am besten bewährt. Falls mit hydrogenolytisch abspaltbaren Schutzgruppen wie z. B. Benzyl gearbeitet wurde, wird diese mit Wasserstoff in Gegenwart von z. B. Raney-Nickel entfernt oder im Falle einer sauer abspaltbaren Schutzgruppe wie z. B. t-Butyl diese mit z. B. Trifluoressigsäure entfernt.

Für die Isolierung der Aminosäure gemäß Formel I (A = OH) wird die Reaktionslösung am geeignetsten mit einer Mineralsäure wie Salzsäure oder Schwefelsäure bis zum neutralen Punkt azidifiziert. Die ausgefallene Aminosäure wird entweder abgesaugt oder mit einem geeigneten Lösungsmittel wie Diethylether, Methylenchlorid, Chloroform oder Toluol extrahiert.

Je nach Art der Carboxylschutzgruppe R kann deren Abspaltung in an sich bekannter Weise sauer oder hydrogenolytisch erfolgen.

Die Herstellung der physiologisch verträglichen Salze erfolgt nach an sich bekannten Methoden durch Umsatz mit anorganischen oder organischen Basen. Zur Salzbildung sind insbesondere geeignet Alkali- und Erdalkalihydroxyde, -carbonate oder -bicarbonate sowie physiologisch verträgliche organische Verbindungen, die eine primäre, sekundäre oder tertiäre Aminogruppe tragen.

Die Erfindung betrifft auch die neuen Zwischenprodukte der Formel IV, V und VI sowie die Verfahren zu deren Herstellung.

Die Verbindungen der Formel I besitzen wertvolle pharmakologische Eigenschaften, insbesondere nootrope. Die nootrope Wirkung der erfindungsgemäßen Verbindungen wurde an Mäusen, die ein Körpergewicht von 20-25 g besaßen, im inhibitory (passive) avoidance test geprüft. Eine modifizierte Form der von J. Kopp, Z. Bodanecky und M.E. Jarvik beschriebenen Testmethode wurde von J. Bures, O. Buresova und J. Huston im "Techniques and Basic Experiments for the Study of Brain and Behavior", Elsevier Scientific Publishers, Amsterdam 1983, beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie (ECS-Amnesie) aufzuheben vermag.

Die Versuche wurden nach modifizierten Testmethoden mit den erfindungsgemäßen Verbindungen durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß der amnestische Effekt bereits mit einer MED (minimal effective dosis) von 0,1-10 mg/kg p.o. aufzuheben ist. Die Vergleichssubstanz hat eine MED von ca. 500-1000 mg/kg p.o..

In Tabelle 1 sind die MED für einige erfindungsgemäße Verbindungen aufgeführt.

Tabelle 1

| erfindungsgemäße Verbindung aus Beispiel | Inhibitory avoidance MED mg/kg p.o. ECS-Amnesie |
|---|---|
| 34 | 1.6 |
| 48 | 50 |
| 35 | 50 |
| 46 | 25 |
| 49 | 25 |
| 47 | 25 |
| 45 | 25 |
| Piracetam Vgl.-Beispiel | 1000 |

Die erfindungsgemäßen Verbindungen sind auf Grund ihrer pharmakologischen Eigenschaften für die Behandlung cognitiver Dysfunktionen unterschiedlicher Genese, wie sie z.B. bei der Alzheimerschen Krankheit oder der Senilen Demenz auftreten, geeignet.
Die Erfindung betrifft daher weiter die Anwendung der erfindungsgemäßen Verbindungen der Formel I bei der Behandlung und Prophylaxe cognitiver Dysfunktionen.

Die neuen Verbindungen können entweder allein oder mit physiologisch verträglichen Hilfs- oder Trägerstoffen vermischt als Arzneimittel angewandt werden. Sie können zu diesem Zweck oral in Dosen von 0,01-30,0 mg/kg/Tag, vorzugsweise 0,01-10,0 mg/kg/Tag, insbesondere 0,05-2,0 mg/kg/Tag oder parenteral (z.B. intravenös, subkutan oder intramuskulär) in Dosen von 0,001-3,0 mg/kg/Tag, vorzugsweise 0,001-1,0 mg/kg/Tag, insbesondere 0,005-0,2 mg/kg/Tag appliziert werden. Die Dosierung kann in schweren Fällen auch erhöht werden. In vielen Fällen genügen jedoch auch geringere Dosen.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95%, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.
Die Wirkstoffe können oral, parenteral, intravenös oder rectal appliziert werden, wobei die orale Applikation bevorzugt ist.
Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige oder ölige Lösungen. Als

inerte Trägerstoffe können z.B. Gummi arabicum, Magnesia, Magnesiumcarbonat, Kaliumphosphat, Milchzucker, Glukose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür geeigneten Substanzen wie Lösungsvermittler, Emulgatoren oder weiteren Hilfsstoffen in Lösung, Suspension oder Emulsion gebracht. Als Lösungmittel kommen z.B. in Frage physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

Beispiel 1
4-{3-[4-(3-Triflourmethylphenyl)-piperazino]-propionamido}-phenylessigsäuremethylester

0,1 Mol Acrylsäure-(4-methoxycarbonylmethyl)-anilid werden zusammen mit 0,15 Mol 4-(3-Trifluormethylphenyl)-piperazin in 80 ml Methylglycol 4 Stunden am Rückfluß erhitzt. Nach beendeter Reaktion wird das Lösungsmittel im Vakuum abdestilliert und der Rückstand kristallisiert.
F: 229° C(HCl)

Beispiel 2
4-{3-[4-(3-Chlorphenyl)-piperazino]-propionamido}-phenylessigsäuremethylester

0,1 Mol Acrylsäure-(4-methoxycarbonylmethyl)-anilid werden mit 0,3 Mol 4-(3-Chlorphenyl)-piperazin 3 Stunden bei einer Temperatur um 160° C belassen. Nach dem Abkühlen wird aus Alkohol kristallisiert.
F: 129° C

Beispiel 3
3-{4-[3-(4-(3-Chlorphenyl)-piperazino)-propionamido]-phenyl} propionsäuremethylester

0,1 Mol Acrylsäure-4[2-(methoxycarbonyl)-ethyl]-anilid werden mit 0,4 Mol 4-(3-Chlorphenyl)-piperazin 4 Stunden bei 180° C gehalten. Nach dem Erkalten führt man das Rohprodukt in das Hydrochlorid über (Alkohol/ Essigestergemisch).
F: 231° C (HCl)

Beispiel 4
3-{4-[3-(4-(3-Trifluormethylphenyl)-piperazino)-propionamido]-phenyl}-propionsäuremethylester.

0,1 Mol 3-Chloropropionsäure-4[2-(methoxycarbonyl)-ethyl]-anilid werden in 100 ml Dimethylformamid zusammen mit 0,15 Mol Kaliumcarbonat und 0,15 Mol 4-(3-Trifluormethyl)-piperazin 4 Stunden bei 85° C gehalten. Nach beendeter Reaktion verteilt man das Reaktionsgemisch zwischen Wasser und Methylenchlorid, extrahiert die wasserlöslichen Bestandteile durch mehrmaliges Waschen mit Wasser, trocknet die organische Phase, engt ein und überführt das Rohprodukt in das Hydrochlorid.
F: 218° C (HCl)

Beispiel 5
4-[3-(4-(3-Trifluormethylphenyl)-piperazino)-propionamido]-zimtsäuremethylester

Aus 0,1 Mol 3-Brompropionsäure-4-[2-(methoxycarbonylvinyl]-anilid und 0,25 Mol 4-(3-Trifluormethylphenyl)-piperazin analog Beispiel 4 ohne Zusatz von Kaliumcarbonat.
F: 247° C (HCl)

Beispiel 6
4-[3-(4-(3-Chlorphenyl)-piperazino)-propionamido]-zimtsäuremethylester.

Aus 4-(3-Brompropionamido)-zimtsäuremethylester und 4-(3-Chlorphenyl)-piperazin entsprechend Beispiel 5
F: 252° C (HCl)

Beispiel 7
4-{4-[3-(4-(3-Chlorphenyl)-piperazino)-propionamido]-phenyl}-buttersäuremethylester

Aus Acrylsäure-4[3-Methoxycarbonyl)-propyl]-anilid und 4-(3-Chlorophenyl)-piperazin entsprechend Beispiel 3.
F: 238° C (HCl)

Beispiel 8

4-{4-[3-(4-(3-Phenylpropyl)-piperazino)-propionamido]-phenyl}-buttersäuremethylester

Aus Acrylsäure-4[3-(Methoxycarbonyl)-propyl]-anilid und 4-(3-Phenylpropyl)-piperazin in DMF entsprechend Beispiel 1.

F: 239° C (2 HCl)

Beispiel 9

4-[3-(4-(3-Phenylpropyl)-piperazino)-propionamido]-zimtsäuremethylester

Aus 4-(3-Chloropropionamido)-zimtsäuremethylester und 4-(3-Phenylpropyl)-piperazin entsprechend Beispiel 4.

F: 252° C (2 HCl)

Beispiel 10

4-[3-(4-Phenylpiperazino)-propionamido]-zimtsäuremethylester

Aus 4-(3-Chlorpropionamido)-zimtsäuremethylester und 4-Phenyl-piperazin entsprechend Beispiel 4.

F: 256° C (HCl)

Beispiel 11

4-{4-[3-(4-(3-Chlorphenyl)-piperazino)-propionamido]-phenyl-}buttersäureethylester

Aus Acrylsäure-4-[3-(ethoxycarbonyl)-propyl]-anilid und 4-(3-Chlorophenyl)-piperazin entsprechend Beispiel 2.

F: 227° C (HCl)

Beispiel 12

4-[3-(4-Phenylpiperidino)-propionamido]-zimtsäuremethylester

Aus 4-(3-Chloropropionamido)-zimtsäuremehtylester und 4-Phenylpiperidin entsprechend Beispiel 4.
F: 257° C (HCl)

Beispiel 13
4-[3-(3-Phenylpropylamino)-propionamido]-zimtsäuremethylester

Aus 4-(3-Chlorpropionamido)-zimtsäuremethylester und 3-Phenylpropylamin entsprechend Beispiel 5.
F: 245° C (HCl)

Beispiel 14
4-{4-[3-(2-(Indolyl-3)-ethylamino)-propionamido]-phenyl}-buttersäuremethylester

Aus Acrylsäure-4-[3-(methoxycarbonyl)-propyl]-anilid und 2-(Indolyl-3)ethylamin entsprechend Beispiel 1.
F: 170° C (HCl)

Beispiel 15
4-{4-[3-(4-(3-Trifluormethylphenyl)-piperazino)-propionamido]-phenyl}-buttersäuremethylester

Aus Acrylsäure-4-[3-(Methoxycarbonyl)-propyl]-anilid und 4-(3-Triflourmethylphenyl)-piperazin entsprechend Beispiel 4.
F: 225° C (HCl)

Beispiel 16
4-{4[3-(4-Phenylpiperazino)-propionamido]-phenyl}-buttersäuremethylester

Aus Acrylsäure-4-[3-Methoxycarbonyl)-propyl]-anilid und 4-Phenylpiperazin entsprechend Beispiel 2.
F: 244° C (HCl)

Beispiel 17

4-{4-[3-(3-Phenylpropylamino)-propionamido]-phenyl}-buttersäuremethylester

Aus Acrylsäure-4-[3-(Methoxycarbonyl)-propyl]-anilid und 3-Phenylpropylamin analog Beispiel 3.

F: 195° C (HCl)

Beispiel 18

4-{4-[3-(4-Phenylpiperidino)-propionamido]-phenyl}-buttersäuremethylester

Aus Acrylsäure-4-[3-(Methoxycarbonyl)-propyl]-anilid und 4-Phenylpiperidin entsprechend Beispiel 1.

F: 244° C (HCl)

Beispiel 19

3-{4-(3-(4-Phenylpiperazino)-propionamido]-phenyl}-propionsäuremethylester

Aus Acrylsäure-4-[2-(Methoxycarbonyl)-ethyl]-anilid und 4-Phenylpiperazin entsprechend Beispiel 3.

F: 247° C (HCl)

Beispiel 20

3{-4-[3-(4-(3-Phenylpropyl)-piperazino)propionamido]-phenyl}-propionsäuremethylester

Acrylsäure-4-[2-(methoxycarbonyl)-ethyl]-anilid und 4-(3-Phenylpropyl)-piperazin entsprechend Beispiel 1.

F: 236° C (2 HCl)

Beispiel 21

3-{4-[3-(3-Phenylpropylamino)-propionamido]-phenyl}-propionsäuremethylester

Aus Acrylsäure-4-[2-methoxycarbonyl)-ethyl]-anilid und 3-Phenylpropylamin analog Beispiel 3.
F: 209° C (HCl)

Beispiel 22
3-{4[3-(4-(3-Chlorphenyl-)-1,2,5,6-tetrahydropyridino)-propionamido]-phenyl}-propionsäuremethylester.

Aus Acrylsäure-4-[2-(methoxycarbonyl)-ethyl]-anilid und 4-(3-Chlorphenyl)-1,2,5,6-tetrahydropyridin entsprechend Beispiel 1.
F: 222° C (HCl)

Beispiel 23
3-{4-[3-(3-(3-Chlorphenyl)-propylamino)-propionamido]-phenyl}-propionsäuremethylester.

Aus Acrylsäure-4-[2-methoxycarbonyl)-ethyl]anilid und 3-(3-Chlorophenyl)-propylamin entsprechend Beispiel 1.
F: 167° C (HCl)

Beispiel 24
3-{4-[3-(4-Phenylpiperidino)-propionamido]-phenyl}-propionsäuremethylester

Aus Acrylsäure-4-[2-(methoxycarbonyl)-ethyl]-anilid und 4-Phenylpiperidin analog Beispiel 3.
F: 254° C (HCl)

Beispiel 25
3-{4-[3-(3-(3-Trifluormethylphenyl)-propylamino)-propionamido]-phenyl}-propionsäuremethylester.

Aus Acrylsäure-4-[2-(methoxycarbonyl)-ethyl]-anilid und 3-(3-Trifluorphenyl)-propylamin analog Beispiel 3.
F: 168° C (HCl)

Beispiel 26
3-{4-[3-(2-(Indolyl-3)-ethylamino)-propionamido]-phenyl}-propionsäuremethylester

Aus Acrylsäure-4[2-(methoxycarbonyl)-ethyl]anilid und 2-(Indolyl-3)-ethylamin analog Beispiel 1.
F: 182° C (HCl)

Beispiel 27
3-{4-[3-(4-(3-Chlorphenyl)-piperazino)-propionamido]-phenyl}-propionsäureamid.
Aus Acrylsäure-4-[2-aminocarbonyl)-ethyl]anilid und 4-(3-Chlorphenyl-piperazin entsprechend Beispiel 3.
F: 185° C

Beispiel 28
3-{4-[3-(4-(3-Trifluormethylphenyl)-piperazino)-propionamido]-phenyl}-propionsäureamid

Aus Acrylsäure-4-[2-(aminocarbonyl)-ethyl]anilid und 4-(3-Trifluormethylphenyl)-piperazin entsprechend Beispiel 3.
F: 168° C

Beispiel 29
3-{4-[3-(4-(3-Chlorphenyl)-piperazino)-propionamido]-phenyl} propionsäureisopropylester.

Aus Acrylsäure-4-[2-(isopropoxycarbonyl)-ethyl]anilid und 4-(3-Chlorphenyl)-piperazin analog Beispiel 2.
F: 237° C (HCl)

Beispiel 30
3-{4-[3-(3-Phenylpropylamino)-propionamido]-phenyl}-propionsäureisopropylester

Aus Acrylsäure-4-[2-(isopropoxycarbonyl)-ethyl]anilid und 3-Phenylpropylamin analog Beispiel 3.
F: 186° (HCl)

Beispiel 31

3-{4-[3-(4-(3-(3-Chlorphenyl)-propyl)-piperazino)-propionamido]-phenyl}-propionsäureisopropylester

Aus Acrylsäure-4-[2-(isopropoxycarbonyl)-ethyl]anilid und 4-[3-(3-Chlorphenyl)-propyl]-piperazin analog Beispiel 1.
F: 230° C (2 HCl)

Beispiel 32

3-{4-[3-(4-(3-Trifluormethylphenyl)-piperazino)-propionamido]-phenyl}-propionsäure

0,01 Mol 3-{4-(3-(4-(3-Trifluormethylphenyl)-piperazino)-propionamido]-phenyl}-propionsäuremethylester werden mit 0,01 Mol KOH in Ethanol 12 Stunden bei Raumtemperatur gehalten. Die Reaktionslösung wird eingeengt, in Wasser aufgenommen und mit Salzsäure auf pH = 7,5 gestellt. Die ausgefallene Aminosäure wird abgesaugt und aus einem Alkohol/Wasser - Gemisch umkristallisiert.
F: 186° C

Beispiel 33

4-{3-[4-(3-Trifluormethylphenyl)-piperazino]-propionamido}-zimtsäure

0,01 Mol 4-{3-[4-(3-Trifluormethylphenyl)-piperazino]-propionamido}-zimtsäuremethylester werden in 50 ml eines Alkohol/Wasser (1:1)-Gemisches mit 0,01 Mol NaOH versetzt. Man hält 4 Stunden bei 50° C und stellt die Lösung nach dem Abkühlen auf pH = 7,5 (Eisessig). Man saugt ab und kristallisiert um.
F: 199° C

Beispiel 34

3-{4-(3-(4-(3-Chlorphenyl)-piperazino)-propionamido]-phenyl} -propionsäure

Aus 3-{4-[3-(4-(3-Chlorphenyl)-piperazino)-propionamido]-phenyl}-propionsäuremethylester analog Beispiel 33.

F: 221° C (HCl)

Beispiel 35

4-{3-[4-(3-Chlorphenyl)-piperazino]-propionamido}-zimtsäure

Aus 4-{3-[4-(3-Chlorphenyl)-piperazino]-propionamido} -zimtsäuremethylester analog Beispiel 33.

F: 263° C (HCl)

Beispiel 36

4-{4-[3-(4-(3-Chlorphenyl)-piperazino)-propionamido]-phenyl}-buttersäure.

Aus 4-{4-[3-(4-(3-Chlorphenyl)-piperazino)-propionamido]-phenyl}-buttersäuremethylester analog Beispiel 33.

F: 208° C

Beispiel 37

4-{4-[3-(4-(3-Phenylpropyl)-piperazino)-propionamido]-phenyl}-buttersäure

Aus 4-{4-[3-(4-(3-Phenylpropyl)-piperazino)-propionamido]-phenyl }-buttersäuremethylester analog Beispiel 32.

F: 214° C (Oxalat)

Beispiel 38

4-{3-[4-Phenylpiperazino]-propionamido}-zimtsäure

Aus 4-[3-(4-Phenylpiperazino)-propionamido]-zimtsäuremethylester analog Beispiel 33.
F: 282° C (HCl)

Beispiel 39

4-[3-(4-(3-Phenylpropyl)-piperazino)-propionamido]-zimtsäure

Aus 4-[3-(4-(3-Phenylpropyl)-piperazino)-propionamido]-zimtsäuremethylester analog Beispiel 33
F: 258° C (HCl)

Beispiel 40

4-{3-(4-Phenylpiperidino)-propionamido}-zimtsäure

Aus 4-{3-[4-Phenylpiperidino]-propionamido-}zimtsäuremethylester analog Beispiel 33.
F: 266° C (HCl)

Beispiel 41

4-[3-(3-Phenylpropylamino)-propionamido]-zimtsäure

Aus 4-[3-(3-Phenylpropylamino)-propionamido]-zimtsäuremethylester entsprechend Beispiel 33.
F: 245° C (HCl)

Beispiel 42

4-{4-[3-(4-(3-Trifluoromethylphenyl)-piperazino)-propionamido]-phenyl}-buttersäure

Aus 4-{4-[3-(4-(3-Trifluormethylphenyl)-piperazino)-propionamido]-phenyl}-buttersäuremethylester analog Beispiel 33.
F: 188° C

Beispiel 43

4-{4-[3-(4-Phenylpiperazino)-propionamido]-phenyl}-buttersäure

Aus 4-{4-[3-(4-Phenylpiperazino)-propionamido]-phenyl}-buttersäuremethylester analog Beispiel 33.

F: 206° C

Beispiel 44

4-{4-[3-(3-Phenylpropylamino)-propionamido]-phenyl}-buttersäure

Aus 4-{4-[3-(3-Phenylpropylamino)-propionamido]-phenyl}-buttersäuremethylester analog Beispiel 33.

F: 172° C

Beispiel 45

4-{4-[3-(4-Phenylpiperidino)-propionamido]-phenyl}-buttersäure

Aus 4-{4-[3-(4-Phenylpiperidino)-propionamido]-phenyl}-buttersäure analog Beispiel 33.

F: 205° C

Beispiel 46

3-{4-[3-(4-Phenylpiperazino)-propionamido]-phenyl}-propionsäure

Aus 3-{4-[3-(4-Phenylpiperazino)-propionamido]-phenyl}-propionsäuremethylester analog Beispiel 33

F: 228° C

Beispiel 47

3-{4-[3-(4-(3-Phenylpropyl)-piperazino)-propionamido]-phenyl}-propionsäure

Aus 3-{4-[3-(4-(3-Phenylpropyl)-piperazino)-propionamido]-phenyl}-propionsäuremethylester analog Beispiel 33.
F: 241° C (2 HCl)

Beispiel 48
3-{4-[3-(3-Phenylpropylamino)-propionamido]-phenyl}-propionsäure

Aus 3-{4-[3-(3-Phenylpropylamino)-propionamido]-phenyl}-propionsäuremethylester analog Beispiel 33.
F: 177° C

Beispiel 49
3-{4-[3-(4-(3-Chlorphenyl)-1,2,5,6-tetrahydropyridino)-propionamido]-phenyl}-propionsäure

Aus 3-{4-[3-(4-(3-Chlorphenyl)-1,2,5,6-tetrahydropyridino)-propionamido]-phenyl}-propionsäurmethylester analog Beispiel 33.
F: 208° C (HCl)

Beispiel 50
3-{4-[3-(3-(3-Chlorphenyl)-propylamino)-propionamido]-phenyl}-propionsäuremethylester analog Beispiel 33.
F: 191° C (HCl)

Beispiel 51
3-{4-[3-(4-Phenylpiperidino)-propionamido]-phenyl}-propionsäure

Aus 3-{4-[3-(4-Phenylpiperidino)-propionamido]-phenyl}-propionsäuremethylester analog Beispiel 33.
F: 212° C

Beispiel 52

3-{4-[3-(2-(Indolyl-3)-ethylamino)-propionamido]-phenyl}-propionsäure

Aus 3-{4-[3-(2-(Indolyl-3)-ethylamino)-propionamido]-phenyl}-propionsäuremethylester analog Beispiel 33.

F: 196° C (HCl)

Beispiel 53

4-{3-[4-(3-Chlorphenyl)-piperazino]-propionamido}-phenylessigsäure

Aus 4-{3-(4-(3-Chlorphenyl)-piperazino]-propionamido}-phenylessigsäuremethylester analog Beispiel 33.

F: 240° C (Acetat)

Beispiel 54

4-{3-[4-(3-Trifluormethylphenyl)-piperazino]-propionamido}-phenylessigsäure

Aus 4-{3-[4-(3-Trifluormethylphenyl)-piperazino]-propionamido}-phenylessigsäuremethylester analog Beispiel 33.

F: 232° C

Beispiel 55

3-{4-[3-(4-(3-Chlorphenyl)-piperazino)-propionamido]-phenyl}-propionsäuremethylester

3-[4-(3-Chlorphenyl)-piperazino]-propionsäure

Zu einer Lösung von 0,05 Mol Acrylsäureethylester in 50 ml Alkohol läßt man eine Lösung von 0,055 Mol 4-(3-Chlorphenyl)-piperazin in 50 ml Alkohol unter Temperaturkontrolle (23 - 28° C) tropfen und rührt

anschließend bei Raumtemperatur 24 h nach. Man fügt anschließend 15 ml einer 10 molaren wäßrigen Lösung von NaOH zu und beläßt weitere 24 h bei Raumtemperatur. Man engt ein, nimmt in 250 ml Wasser auf und stellt mit 10 n wäßriger Salzsäure neutral und extrahiert die freie Aminosäure mit Butylacetat. Nach dem Trocknen und Einengen der organischen Phase verbleibt die rohe 3-[4-(3-Chlorphenyl)-piperazino]-propionsäure, die ohne weitere Reinigung umgesetzt wird.

3-{4-[3-(4-(3-Chlorphenyl)-piperazino)-propionamido]-phenyl}-propionsäuremethylester

In 40 ml Tetrahydrofuran werden 0,03 Mol 3-[4-(3-Chlorphenyl)-piperazino]-propionsäure mit 0,062 Mol Triethylamin unter Eiskühlung versetzt. Dann gibt man ebenfalls unter Eiskühlung 0,03 Mol Chlorameisensäureethylester in 40 ml Tetrahydrofuran zu und rührt noch 20 Minuten bei 10°C nach. Anschließend wird bei 5 - 8° C eine Lösung von 0,03 Mol 3-(4-Aminophenyl)-propionsäuremethylester in 40 ml Tetrahydrofuran zugegeben und fünf Stunden bei Raumtemperatur nachgerührt. Man engt ein, verteilt das Rohprodukt zwischen Methylenchlorid und Wasser, trocknet und überführt den nach dem Einengen erhaltenen rohen Ester in das Hydrochlorid (Alkohol/Essigester)
F: 230 - 231° C (HCl).

Patentansprüche

I. Substituierte Aminopropionsäureamide der Formel I

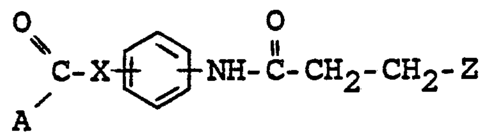

(I)

in der

A Hydroxy, Amino, Alkyl- und Dialkylamino mit jeweils 1-4 C-Atomen im Alkylteil, Alkoxy mit 1-4 C-Atomen im Alkylteil, wobei die genannten Alkylgruppen geradkettig oder verzweigt sein können,

X Alkylen mit 1-4 C-Atomen oder Alkenylen mit 2-4 C-Atomen, wobei die genannten Alkylgruppen geradkettig oder verzweigt sein können und

Z eine Aminogruppe der Formel II

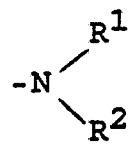

(II)

ist, worin

$R_1$ Wasserstoff und

$R_2$ Aryl-$C_1$-$C_4$-alkyl, wobei die Alkylgruppe geradkettig oder verzweigt ist und Aryl, Phenyl, Pyridyl, Indolyl, Benzofuranyl, Benzothienyl, Pyrrolyl, Furanyl oder Thienyl bedeutet, wobei die Arylreste mit Halogen, Methyl oder 1-, 2- oder 3-fach halogensubstituiertem Methyl mono- oder disubstituiert sein können,

bedeutet

oder worin $R_1$ und $R_2$ zusammen mit dem Stickstoffatom einen gesättigten oder ungesättigten 5- oder 6-gliedrigen heterocyclischen Ring bilden, der mit Phenyl oder Phenyl-alkyl mit 1-4 C-Atomen im Alkylteil

substituiert ist, wobei die Phenylreste ihrerseits mit Halogen, Methyl oder 1-, 2- oder 3-fach halogensubstituiertem Methyl mono- oder disubstituiert sein können, wobei im Falle des 6-gliedrigen Ringes der Phenyl- oder Phenyl-$C_1$-$C_4$-alkylrest über ein zweites, im 6-Ring befindliches Stickstoffatom gebunden sein kann sowie deren physiologisch verträglichen Salze.

2. Substituierte Aminopropionsäureamide der Formel I in denen

A Hydroxy, Amino, Alkoxy mit 1-4 C-Atomen im Alkylteil, wobei die genannte Alkylgruppe geradkettig oder verzweigt sein kann

X Alkylen mit 1-4 C-Atomen oder Alkenylen mit 2 C-Atomen, wobei die genannte Alkylgruppe geradkettig oder verzweigt sein kann

Z eine Aminogruppe der Formel II

ist, worin

$R_1$ Wasserstoff und

$R_2$ Aryl-$C_1$-$C_4$-Alkyl, wobei die Alkylgruppe geradkettig oder verzweigt ist und Aryl, Phenyl, Pyridyl, Indolyl, Benzofuranyl, Benzothienyl, Pyrrolyl, Furanyl oder Thienyl bedeutet, wobei die Arylreste mit Fluor, Chlor, Brom, Methyl oder Trifluormethyl monosubstituiert sein können, bedeutet

oder worin $R_1$ und $R_2$ zusammen mit dem Stickstoffatom einen gesättigten oder ungesättigten 5- oder 6-gliedrigen heterozyklischen Ring bilden, der mit Phenyl oder Phenylalkyl mit 1 bis 4 C-Atomen im Alkylteil substituiert ist, wobei die Phenylreste ihrerseits mit Fluor, Chlor, Brom, Methyl oder Trifluormethyl

monosubstituiert sein können und wobei, im Falle des 6-gliedrigen Ringes, der Phenyl- oder Phenyl-$C_1$-$C_4$-alkylrest über ein zweites im 6-Ring befindliches Stickstoffatom gebunden sein kann

sowie deren physiologisch verträglichen Salze.

3. Substituierte Aminopropionsäureamide der Formel Ia

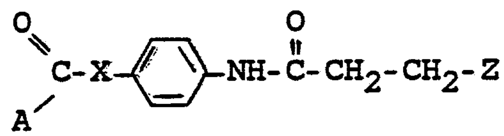

Ia

in der

A Hydroxy

X Methylen oder Ethylen

Z eine Aminogruppe der Formel II

ist, worin

$R_1$ Wasserstoff

$R_2$ Aryl-$C_1$-$C_3$-Alkyl, wobei die Alkylkette geradkettig ist und Aryl Phenyl oder Indolyl bedeutet, wobei die Phenylreste gegebenenfalls mit Chlor oder Trifluormethyl monosubstituiert sind, bedeutet

oder worin $R_1$ und $R_2$ zusammen mit dem Stickstoffatom einen gesättigten oder 1-fach ungesättigten, 6-gliedrigen Ring bilden, der gegebenenfalls in 4-Stellung ein zweites, im Ring befindliches Stickstoffatom besitzt und in 4-Stellung mit Phenyl substituiert ist, wobei dieser Phenylrest seinerseits mit Chlor oder Trifluormethyl monosubstituiert sein kann

sowie deren physiologisch verträgliche Salze.

4. Verfahren zur Herstellung von substituierten Aminopropionsäureamiden der Formel I gemäß Anspruch 1, dadurch gekennzeichnet, daß man

a) Amine der Formel III

H - Z (III)

oder deren quaternäre Salze, wobei Z die in Anspruch 1 zu Formel I angegebene Bedeutung hat, mit Acrylsäureaniliden der Formel IV,

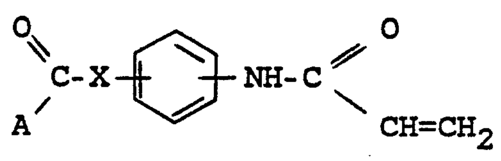

(IV)

worin A und X die in Anspruch 1 zu Formel I angegebenen Bedeutungen haben, und worin, falls A Hydroxy ist, die vorhandene freie Carboxyfunktion gegebenenfalls geschützt ist, umsetzt oder

b) das Amin der Formel III mit einem Propionsäureanilid der Formel V,

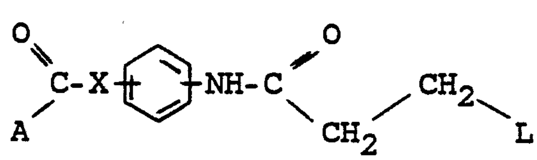

(V)

worin A und X die in Anspruch 1 zu Formel I angegebenen Bedeutungen haben und L einen durch nukleophile Substitution ersetzbaren Rest darstellt und worin, falls A Hydroxy ist, die vorhandene freie Carboxyfunktion gegebenenfalls geschützt ist, umsetzt, oder

c) Aminopropionsäurederivate der Formel VI,

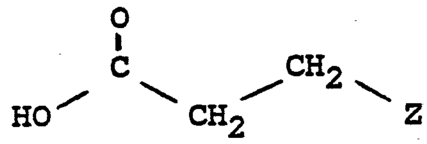

(VI)

worin Z die in Anspruch 1 zu Formel I angegebene Bedeutung hat

mit Anilinderivaten der Formel VII

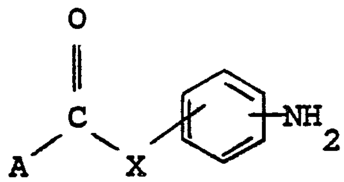

(VII)

umsetzt, wobei A und X die in Anspruch 1 zu Formel I angegebenen Bedeutungen haben und worin, falls A Hydroxy ist, die vorhandene freie Carboxyfunktion gegebenenfalls geschützt ist

und in den nach Verfahrensvariante a), b) oder c) hergestellten Verbindungen die gegebenenfalls vorhandene Schutzgruppe abspaltet und die Reaktionsprodukte gegebenenfalls in ihre physiologisch verträglichen Salze überführt.

5. Verfahren nach Anspruch 3, dadurch gekennzeichnet, daß in den Verfahrensvarianten a), b) oder c) die 1,4-substituierten Phenyle der Verbindungen IV, V oder VII eingesetzt werden.

6. Arzneimittel, dadurch gekennzeichnet, daß es eine Verbindung der Formel I gemäß Anspruch 1 oder ein physiologisch verträgliches Salz dieser Verbindung in einer therapeutisch wirksamen Menge, gegebenenfalls zusammen mit üblichen pharmazeutischen Hilfs- oder Trägerstoffen enthält.

7. Arzneimittel nach Anspruch 6, dadurch gekennzeichnet,

daß es nootrop wirksame Mengen einer Verbindung nach Anspruch 1 enthält.

8. Verwendung einer Verbindung der Formel I gemäß Anspruch 1 oder eines physiologisch verträglichen Salzes dieser Verbindung zur Herstellung eines Arzneimittels.

9. Verwendung einer Verbindung der Formel I gemäß Anspruch 1 oder eines physiologisch verträglichen Salzes dieser Verbindung als Nootropikum.

10. Verbindungen der Formel I gemäß Anspruch 1 zur Verwendung als Nootropikum.

11. Acrylsäureanilide der Formel IV gemäß Anspruch 4, wobei A und X die in Anspruch 1 zu Formel I angegebenen Bedeutungen haben.

12. Propionsäureanilide der Formel V gemäß Anspruch 4, wobei A und X die in Anspruch 1 zu Formel I und L die in Anspruch 4 zu Formel V angegebenen Bedeutungen haben.

13. Aminopropionsäurederivate der Formel VI gemäß Anspruch 4, wobei Z die in Anspruch 1 zu Formel I angegebene Bedeutung hat.

Patentansprüche für ES, GR

1. Verfahren zur Herstellung von substituierten Aminopropionsäureamiden der Formel I

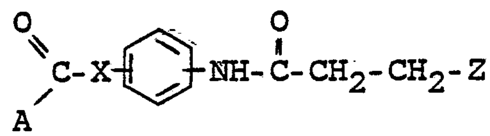

(I)

in der

A Hydroxy, Amino, Alkyl- und Dialkylamino mit jeweils 1-4 C-Atomen im Alkylteil, Alkoxy mit 1-4 C-Atomen im Alkylteil, wobei die genannten Alkylgruppen geradkettig oder verzweigt sein können,

X Alkylen mit 1-4 C-Atomen oder Alkenylen mit 2-4 C-Atomen, wobei die genannten Alkylgruppen geradkettig oder verzweigt sein können und

Z eine Aminogruppe der Formel II

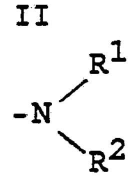

(II)

ist, worin

$R_1$ Wasserstoff und

$R_2$ Aryl-$C_1$-$C_4$-alkyl, wobei die Alkylgruppe geradkettig oder verzweigt ist und Aryl, Phenyl, Pyridyl, Indolyl, Benzofuranyl, Benzothienyl, Pyrrolyl, Furanyl oder Thienyl bedeutet, wobei die Arylreste mit Halogen, Methyl oder 1-, 2- oder 3-fach halogensubstituiertem Methyl mono- oder disubstituiert sein können,

bedeutet

oder worin $R_1$ und $R_2$ zusammen mit dem Stickstoffatom einen gesättigten oder ungesättigten 5- oder 6-gliedrigen heterocyclischen Ring bilden, der mit Phenyl oder Phenyl-alkyl mit 1-4 C-Atomen im Alkylteil

substituiert ist, wobei die Phenylreste ihrerseits mit Halogen, Methyl oder 1-, 2- oder 3-fach halogensubstituiertem Methyl mono- oder disubstituiert sein können, wobei im Falle des 6-gliedrigen Ringes der Phenyl- oder Phenyl-$C_1$-$C_4$-alkylrest über ein zweites, im 6-Ring befindliches Stickstoffatom gebunden sein kann,

dadurch gekennzeichnet, daß man

a) Amine der Formel III

H - Z (III)

oder deren quaternäre Salze, wobei Z die zu Formel I angegebene Bedeutung hat, mit Acrylsäureaniliden der Formel IV,

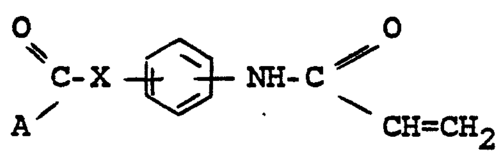

(IV)

worin A und X die zu Formel I angegebenen Bedeutungen haben, und worin, falls A Hydroxy ist, die vorhandene freie Carboxyfunktion gegebenenfalls geschützt ist, umsetzt oder

b) das Amin der Formel III mit einem Propionsäureanilid der Formel V,

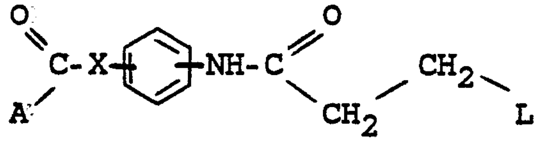

(V)

worin A und X die zu Formel I angegebenen Bedeutungen haben und L einen durch nukleophile Substitution ersetzbaren Rest darstellt und worin, falls A Hydroxy ist, die vorhandene freie Carboxyfunktion gegebenenfalls geschützt ist, umsetzt, oder

c) Aminopropionsäurederivate der Formel VI,

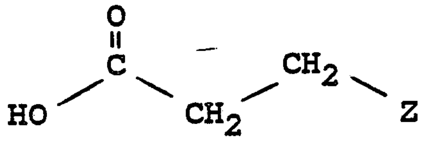

(VI)

worin Z die zu Formel I angegebene Bedeutung hat

mit Anilinderivaten der Formel VII

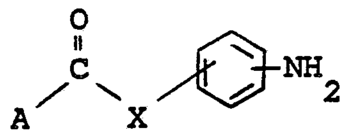

(VII)

umsetzt, wobei A und X die in zu Formel I angegebenen Bedeutungen haben und worin, falls A Hydroxy ist, die vorhandene freie Carboxyfunktion gegebenenfalls geschützt ist

und in den nach Verfahrensvariante a), b) oder c) hergestellten Verbindungen die gegebenenfalls vorhandene Schutzgruppe abspaltet und die Reaktionsprodukte gegebenenfalls in ihre physiologisch verträglichen Salze überführt.

2. Verfahren nach Anspruch 1, dadurch gekennzeichnet, daß man substituierte Aminopropionsäureamide der Formel I gemäß Anspruch 1, in denen

A Hydroxy, Amino, Alkoxy mit 1-4 C-Atomen im Alkylteil, wobei die genannte Alkylgruppe geradkettig oder verzweigt sein kann

X Alkylen mit 1-4 C-Atomen oder Alkenylen mit 2 C-Atomen, wobei die genannte Alkylgruppe geradkettig oder verzweigt sein kann

Z eine Aminogruppe der Formel II

ist, worin

$R_1$ Wasserstoff und

$R_2$ Aryl-$C_1$-$C_4$-Alkyl, wobei die Alkylgruppe geradkettig oder verzweigt ist und Aryl, Phenyl, Pyridyl, Indolyl, Benzofuranyl, Benzothienyl, Pyrrolyl, Furanyl oder Thienyl bedeutet, wobei die Arylreste mit Fluor, Chlor, Brom, Methyl oder Trifluormethyl monosubstituiert sein können, bedeutet

oder worin $R_1$ und $R_2$ zusammen mit dem Stickstoffatom einen gesättigten oder ungesättigten 5- oder 6-gliedrigen heterozyklischen Ring bilden, der mit Phenyl oder Phenylalkyl mit 1 bis 4 C-Atomen im Alkylteil substituiert ist, wobei die Phenylreste ihrerseits mit Fluor, Chlor, Brom, Methyl oder Trifluormethyl monosubstituiert sein können und wobei, im Falle des 6-gliedrigen Ringes, der Phenyl- oder Phenyl-$C_1$-$C_4$-alkylrest über ein zweites im 6-Ring befindliches Stickstoffatom gebunden sein kann,

herstellt und diese Verbindungen gegebenenfalls in ihre physiologisch verträglichen Salze überführt.

3. Verfahren nach Anspruch 1, dadurch gekennzeichent, daß man substituierte Aminopropionsäureamide der Formel Ia

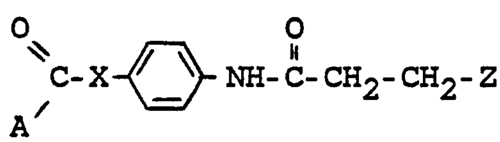

Ia

in der

A Hydroxy

X Methylen oder Ethylen

Z eine Aminogruppe der Formel II

ist, worin

$R_1$ Wasserstoff

$R_2$ Aryl-$C_1$-$C_3$-Alkyl, wobei die Alkylkette geradkettig ist und Aryl Phenyl oder Indolyl bedeutet, wobei die Phenylreste gegebenenfalls mit Chlor oder Trifluormethyl monosubstituiert sind, bedeutet

oder worin $R_1$ und $R_2$ zusammen mit dem Stickstoffatom einen gesättigten oder 1-fach ungesättigten, 6-gliedrigen Ring bilden, der gegebenenfalls in 4-Stellung ein zweites, im Ring befindliches Stickstoffatom besitzt und in 4-Stellung mit Phenyl substituiert ist, wobei dieser Phenylrest seinerseits mit Chlor oder Trifluormethyl monosubstituiert sein kann,

herstellt und diese Verbindungen gegebenenfalls in ihre physiologisch verträglichen Salze überführt.

4. Verfahren nach Anspruch 1 oder 2, dadurch gekennzeichnet, daß in den Verfahrensvarianten a), b) oder c) die 1,4-substituierten Phenyle der Verbindungen IV, V oder VII eingesetzt werden.

5. Arzneimittel, dadurch gekennzeichnet, daß es eine Verbindung der Formel I erhältlich gemäß Anspruch 1 oder ein physiologisch verträgliches Salz dieser Verbindung in einer therapeutisch wirksamen Menge, gegebenenfalls zusammen mit üblichen pharmazeutischen Hilfs- oder Trägerstoffen enthält.

6. Arzneimittel nach Anspruch 5, dadurch gekennzeichnet, daß es nootrop wirksame Mengen einer Verbindung erhältlich nach Anspruch 1 enthält.

7. Verwendung einer Verbindung der Formel I erhältlich gemäß Anspruch 1 oder eines physiologisch verträglichen Salzes dieser Verbindung zur Herstellung eines Arzneimittels.

8. Verwendung einer Verbindung der Formel I erhältlich gemäß Anspruch 1 oder eines physiologisch verträglichen Salzes dieser Verbindung als Nootropikum.

9. Verbindungen der Formel I oder deren physiologisch verträgliche Salze erhältlich nach Anspruch 1.

10. Verbindungen der Formel I erhältlich gemäß Anspruch 1 zur Verwendung als Nootropikum.

11. Acrylsäureanilide der Formel IV gemäß Anspruch 1, wobei A und X die in Anspruch 1 zu Formel I angegebenen Bedeutungen haben.

12. Propionsäureanilide der Formel V gemäß Anspruch 1, wobei A und X die in Anspruch 1 zu Formel I und L die in Anspruch 1 zu Formel V angegebenen Bedeutungen haben.

13. Aminopropionsäurederivate der Formel VI gemäß Anspruch 1, wobei Z die in Anspruch 1 zu Formel I angegebene Bedeutung hat.